# EUROPEAN PATENT APPLICATION

(11) **EP 4 245 858 A1**
(43) Date of publication of application: **20.09.2023**
(21) Application number: 22723000.0
(22) Date of filing: 17.01.2022
(51) Int. Cl.: C12Q 1/68, C12N 15/11

(54) **METHOD FOR DETECTING HUMAN MICROSATELLITE INSTABILITY SITE, AND USE THEREOF**

(30) Priority: 16.11.2020 CN 202011280938
(71) Applicant: Huang, Xin, Shanghai 200127 (CN); Jiang, Ying, Shanghai 200127 (CN)
(72) Inventor: Huang, Xin, Shanghai 200127 (CN); Jiang, Ying, Shanghai 200127 (CN)
(74) Representative: Dehns
(86) International application number: PCT/CN2022/072414
(87) International publication number: WO 2022/100766

(57) **Abstract**

Provided is a method for detecting a human microsatellite instability (MSI) site, and a use thereof. Disclosed are a primer, a probe, and a detection system used for detecting an MSI. The present method and kit may be used for detecting whether MSI-H is present in a tumor patient, and provide medication guidance or provide risk assessment guidance according to a detection result. The present invention has advantages such as high sensitivity, high specificity, and strong anti-interference capability.

## Description

### Technical field

The invention relates to the field of medicine and biotechnology, in particular to a method for detecting unstable sites of human microsatellites and applications thereof.

### Background

DNA mismatch repair (MMR) gene mutation or promoter methylation is one of the important causes of cancers. The protein encoded by the MMR gene monitors base mismatches during DNA replication to avoid errors. Mismatch repair proteins include two families, MutS and MutL, the former including MSH2/MSH3 and MSH6, etc., and the latter including MLH1, MLH3, PMS1 and PMS2. MSH2 and MLH1 form complexes with their homologous mismatch repair proteins respectively to exert their function. For example, MSH2 forms complexes MutSα and MutSβ with MSH6 and MSH3, respectively; MLH1 forms the complexes MutLα, MutLβ or MutLy with PMS2, PMS1 or MLH3, respectively. The steps of MutS α or MutSβ for repairing mismatch bases are as follows: after identifying the mismatch bases, binding to DNA bases, then binding to MutL, activating ATPase, hydrolyzing mismatched bases, and activating endonuclease I, excising and repairing mismatched bases.

Microsatellite is a short series of repeats, with each unit being 1-6bp in length. It is widely present in the genomes of prokaryotic and eukaryotic organisms and has high genetic stability. However, when the function of the cell's mismatch repair gene is abnormal, the number of repeat nucleotides of the daughter cell microsatellite can increase or decrease, resulting in a change in the length of the microsatellite, and such phenomenon is called microsatellite stability (MSI).

The mutation of mismatch repair gene or promoter methylation can lead to the reduction of DNA mismatch repair function, leading to MSI. Many important genes related to growth regulation, such as type II TGF-β, IGF2R, PTEN, BAX, contain microsatellites in their coding regions or promoter regions. MSI caused by mismatch repair abnormalities can cause missense mutations or frameshift mutations in the replication process of these genes, making such replication errors accumulate continuously and become an important factor leading to the occurrence of tumors. Therefore, MSI can be used as a molecular marker for tumors. At present, high-frequency MSI has been found in a variety of tumors, such as colorectal cancer, gastric cancer, small intestine cancer, endometrial cancer, etc.

In addition, although there are some MSI detection methods, the existing MSI detection technology is cumbersome, the samples are easily to be contaminated by each other, and the detection takes a long time. The positive interpretation is highly subjective, the requirement on the type and quality of the sample are high, in most situations, tumor and adjacent tissue samples are needed at the same time, and the proportion of tumor cells in tumor tissue samples is not less than 20%.

In summary, there is an urgent need in the art to develop a method for detecting tumor MSI with high sensitivity, high specificity, eliminating the possibility of sample contamination and strong anti-interference ability.

### Summary of the invention

The purpose of the present invention is to provide a method for detecting tumor MSI with high sensitivity, high specificity, eliminating the possibility of sample contamination and strong anti-interference ability.

The invention also provides a detection method for human micro satellite instability (MSI) site and its application.

In the first aspect of the invention, it is provided a use of a human Microsatellite Instability (MSI) site detection reagent for the preparation of a diagnostic reagent or kit for the diagnosis of MSI-related diseases and/or for the prognosis of MSI-related diseases; wherein the MSI site is selected from one or more sites in the following group A:
(Z1) chr3:30650236-30650508;
(Z2) chr11:106739898-106740117;
(Z3) chr16:18841298-18841518;
(Z4) chr17:19411505-19411722;
(Z5) chr20:62921533-62921750;
(Z6) chr2:47408320-47408461;
(Z7) chr2:147906719-147906938;
(Z8) chr6:142407071-142407290;
(Z9) chr14:93268657-93268877;
(Z10) chr20:47779916-47780134;
(Z11) chr7:143306180-143306440;
(Z12) chr1:201819424-201819543;
(Z13) chr1:201771449-201771558;
(Z14) chr2:61827581-61827677;
(Z15) chr2:147857500-147857584;
(Z16) chr4:82821524-82821646;
(Z17) chr5:172998578-172998712;
(Z18) chr6:142337918-142338138;
(Z19) chr14:93244766-93244900;
(Z20) chr15:45593287-45593508;
(Z21) chr15:33349068-33349160;
(Z22) chr15:33764931-33765150;
(Z23) chr16:18854033-18854164_{∘}

In another preferred example, the detection reagent is selected from the following group: a primer, probe, guild RNA (for CRISPR), chip, and a combination thereof.

In another preferred embodiment, the MSI site is selected from the group consisting of:
(Z1) chr3:30650236-30650508;
(Z2) chr11:106739898-106740117;
(Z3) chr16:18841298-18841518;
(Z4) chr17:19411505-19411722;
(Z5) chr20:62921533-62921750;
(Z6) chr2:47408320-47408461;
(Z7) chr2:147906719-147906938;
(Z8) chr6:142407071-142407290;
(Z9) chr14:93268657-93268877;
(Z10) chr20:47779916-47780134;
(Y1) any combinations of Z1-Z10.

In another preferred embodiment, the MSI site comprises at least 1, at least 2, at least 3, at least 4, or 5 selected from Z1-Z7.

In another preferred embodiment, the MSI site includes Z2, Z3, Z4, Z5 and Z7.

In another preferred embodiment, the MSI site comprises at least 1, at least 2, and at least 3 sites selected from Z1-Z10.

In another preferred embodiment, the MSI site further comprises an additional MSI site in addition to Z1 ~ Z10.

In another preferred embodiment, the MSI site comprises (a) one or more (e.g., 2, 3, 4, or 5) sites selected from Z2, Z3, Z4, Z5 and Z7; and (b) additional MSI sites in addition to Z2, Z3, Z4, Z5 and Z7.

In another preferred embodiment, the MSI site comprises (a) Z2, Z3, Z4, Z5 and Z7; and (b) an additional MSI site in addition to Z2, Z3, Z4, Z5, and Z7.

In another preferred embodiment, the additional MSI site is selected from the following group: BAT-26, BAT-25, MONO-27, NR-21, NR-24, D5S346, D2S123, D17S250, and a combination thereof.

In another preferred embodiment, the MSI-related disease is a tumor or cancer.

In another preferred embodiment, the tumor or cancer is selected from the following group: colorectal cancer, endometrial cancer, uterine sarcoma, gastric cancer, small intestine cancer, cervical cancer, liver cancer, esophageal cancer, pancreatic cancer, ovarian cancer, gallbladder cancer, testicular cancer, prostate cancer, fallopian tube cancer, vulvar cancer, adrenal cortex cancer, primary abdominal tumor, cholangiocarcinoma, breast cancer, neuroendocrine tumor, thymic cancer, thyroid cancer, small cell lung cancer, primary unknown tumor, etc

In another preferred embodiment, the diagnostic reagent or kit is used for a detection selected from the following group: serum detection, plasma detection, cell detection, tissue sample detection.

In another preferred embodiment, the detection is a PCR detection or sequencing detection; preferably, digital PCR (ddPCR) detection.

In another preferred embodiment, the detection includes a single detection or multiple detection (e.g., n-multiple detection, wherein n is any positive integer of 2-20, preferably n is 3-15, preferably 5-10).

In another preferred embodiment, the multiple detection comprises: multiplex amplification is performed in a reaction system, followed by detection.

Preferably, "followed by detection" includes fluorescence detection, capillary electrophoresis, sequencing, and a combination thereof.

In the second aspect of the invention, a reagent is provided for the detection of human Microsatellite Instability (MSI) sites selected from sites 1 ~ 10 in chromosome hg38 (SEQ ID NO.: 1 ~ 10); wherein the reagent is selected from the following group:
(a) The first primer pair for detecting MSI at chr3 site 1(SEQ ID NO.:1), where the first primer pair comprises primers shown in SEQ ID NO.: 11 and 12;
(b) The second primer pair for detecting MSI at chr11 site 2(SEQ ID NO.:2), in which the second primer pair comprises primers shown in SEQ ID NO.: 19 and 20;
(c) The third primer pair for detecting MSI at chr16 site 3 (SEQ ID NO.:3), in which the third primer pair comprises primers shown in SEQ ID NO.:23 and 24;
(d) The fourth primer pair for detecting MSI at chr17 site 4 (SEQ ID NO.:4), where the fourth primer pair comprises the primers shown in SEQ ID NO.:25 and 26;
(e) The fifth primer pair for detecting MSI at chr20 site 5(SEQ ID NO.:5), where the fifth primer pair comprises primers shown in SEQ ID NO.:29 and 30;
(f) The sixth primer pair for detecting MSI at chr2 site 6(SEQ ID NO.:6), where the sixth primer pair comprises primers shown in SEQ ID NO.:13 and 14;
(g) The seventh primer pair for detecting MSI at chr2 site 7(SEQ ID NO.:7), where the seventh primer pair comprises primers shown in SEQ ID NO.:15 and 16;
(h) The eighth primer pair for detecting MSI at chr6 site 8 (SEQ ID NO.:8), where the eighth primer pair comprises the primers shown in SEQ ID NO.:17 and 18;
(i) The ninth primer pair for detecting MSI at chr14 site 9 (SEQ ID NO.:9), where the fourth primer pair comprises the primers shown in SEQ ID NO.:21 and 22;
(j) The tenth primer pair for detecting MSI at chr20 site 10(SEQ ID NO.:10), where the tenth primer pair comprises primers shown in SEQ ID NO.:27 and 28;
any combination of (a) to (j) above.

In another preferred embodiment, the sequence of site 1 is located at chr3:30650236-30650508.

In another preferred embodiment, the sequence of site 2 is located at chr11:106739898-106740117.

In another preferred embodiment, the sequence of site 3 is located at chr16:18841298-18841518.

In another preferred embodiment, the sequence of site 4 is located at chr17:19411505-19411722.

In another preferred embodiment, the sequence of site 5 is located at chr20:62921533-62921750.

In another preferred embodiment, the sequence of site 6 is located at chr2:47408320-47408461.

In another preferred embodiment, the sequence of site 7 is located at chr2:147906719-147906938.

In another preferred embodiment, the sequence of site 8 is located at chr16:142407071-142407290.

In another preferred embodiment, the sequence of site 9 is located at chr2:93268657-93268877.

In another preferred embodiment, the sequence of site 10 is located at chr20:47779916-47780134.

In another preferred embodiment, the preferred sites for the detection of human Microsatellite Instability (MSI) are sites 2, 3, 4, 5 and 7.

In another preferred embodiment, the kit further comprises:
(a1) The first probe used in combination with the first primer pair, wherein the first probe is selected from the following group: a probe as shown in SEQ ID NO.: 31, a probe as shown in SEQ ID NO.: 32 or a combination thereof; and/or
(b1) The second probe used in combination with the second primer pair, wherein the second probe is selected from the following group: a probe as shown in SEQ ID NO.: 39, a probe as shown in SEQ ID NO.: 40 or a combination thereof; and/or
(c1) The third probe used in combination with the third primer pair, wherein the third probe is selected from the following group: a probe as shown in SEQ ID NO.: 43, a probe as shown in SEQ ID NO.: 44 or a combination thereof; and/or
(d1) The fourth probe used in combination with the fourth primer pair, wherein the fourth probe is selected from the following group: a probe as shown in SEQ ID NO.: 45, a probe as shown in SEQ ID NO.: 46 or a combination thereof; and/or
(e1) The fifth probe used in combination with the fifth primer pair, wherein the fifth probe is selected from the following group: a probe as shown in SEQ ID NO.: 49, a probe as shown in SEQ ID NO.: 50 or a combination thereof; and/or
(f1) The sixth probe used in combination with the sixth primer pair, wherein the sixth probe is selected from the following group: a probe as shown in SEQ ID NO.: 33, a probe as shown in SEQ ID NO.: 34 or a combination thereof; and/or
(g1) The seventh probe used in combination with the seventh primer pair, wherein the seventh probe is selected from the following group: a probe as shown in SEQ ID NO.: 35, a probe as shown in SEQ ID NO.: 36 or a combination thereof; and/or
(h1) The eighth probe used in combination with the eighth primer pair, wherein the eighth probe is selected from the following group: a probe as shown in SEQ ID NO.: 37, a probe as shown in SEQ ID NO.: 38 or a combination thereof; and/or
(i1) The ninth probe used in combination with the ninth primer pair, wherein the ninth probe is selected from the following group: a probe as shown in SEQ ID NO.: 41, a probe as shown in SEQ ID NO.: 42 or a combination thereof; and/or
(j 1) The tenth probe used in combination with the tenth primer pair, wherein the tenth probe is selected from the following group: a probe as shown in SEQ ID NO.: 47, a probe as shown in SEQ ID NO.: 48 or a combination thereof.

In another preferred embodiment, the first probe to the tenth probe is a single-stranded nucleic acid probe.

In another preferred embodiment, the structure of the first probe to the tenth probe (5'-3') is shown in formula I:
L1-L2-L3 I

Wherein,
L1 is a fluorescence group and L3 is a quenching group; or L3 is a fluorescence group and L1 is a quenching group;
L2 is a specific complementary nucleic acid sequence of nucleotides;
"-" is a chemical bond, a connecting group, or a linker of one or three nucleotides.

In another preferred embodiment, the specific nucleic acid sequence of L2 specifically targets site 1 to site 10 in hg38.

In another preferred embodiment, the L2 comprises a lock nucleotide modification.

In another preferred embodiment, the sequence of L2 is selected from the group consisting of:
CACCAGGCTTTTTTTTTTCCTTCA (SEQ ID NO.: 31);
CAGCATCTTCCAGAATAAAGTC (SEQ ID NO.: 32);
TTGAGAGGCACTTTTTTTTTTTTTTTTTTTTGAGATG (SEQ ID NO.: 39);
AGTCTTGCTCTGTCACC (SEQ ID NO.: 40);
TTGAGGTACTTTTTTTTTTTTTTTTTTTTTGAGACAC (SEQ ID NO.: 43);
TTGCTCTGTCGCCCATGCT (SEQ ID NO.: 44);
TCTAAATCTTTTTTTTTTTTTTTTTTCTCCAAGGAGAG (SEQ ID NO.: 45);
AAACAGAGCTTCATGGGAAAC (SEQ ID NO.: 46);
CAAATTGGAGCTGCAGCCAATTTTTTTTTTTTTTTTTT (SEQ ID NO.: 49);
CCTGGGCGACAGAGACCC (SEQ ID NO.: 50);
TAATGTACTTTTTTTTTTTTTAAGG (SEQ ID NO.: 33);
CATGTAATATCTCAAATCT (SEQ ID NO.: 34);
TCAAGTCCATTTTTTTTTTTTTTTTTTTTTAACTTTA (SEQ ID NO.: 35);
ATACATGTGCAGAACATGCAGGT (SEQ ID NO.: 36);
ATGCTTCCTTTTTTTTTTTTTTTTTTTTAAAGAGACG (SEQ ID NO.: 37);
CATTCTCCTGTAAATTAAC (SEQ ID NO.: 38);
ACTTAGACCTTTTTTTTTTTTTTTTTTTTTGAGACAGA (SEQ ID NO.: 41);
TTGCTCAGCCGCCCAGG (SEQ ID NO.: 42);
CCCTAGTTGACTTTTTTTTTTTTTTTTTTTGAGACAGA (SEQ ID NO.: 47);
TGACTCTGTTGCCCAGGCTG (SEQ ID NO.: 48).

In another preferred embodiment, the fluorophore is independently located at the 5' end, 3' end and/or middle of the nucleic acid probe.

In another preferred embodiment, the fluorescent group and the quenching group are each independently located at the 5' end, the 3' end, and the middle of the nucleic acid probe.

In another preferred embodiment, the fluorescent group comprises a fluorescent group crosslinked to a DNA probe.

In another preferred embodiment, the fluorescent group is selected from the following groups: FAM, VIC, HEX, FITC, BODIPY-FL, G-Dye100, FluorX, Cy3, Cy5, Texas Red, and a combination thereof.

In another preferred embodiment, the quenching group is selected from the following groups: DABCYL, TAMRA, BHQ 1, BHQ 2, BHQ3, MGB, BBQ-650, TQ1-TQ6, QSY 7 carboxylic acid, TQ7, eclipse, and a combination thereof.

In a third aspect, the present invention provided a kit comprising the reagent for the detection ofg MSI sites of the second aspect of the present invention.

In another preferred example, the MSI site is selected from one or more sites of group A (i.e., one or more of Z1 ~ Z23), and preferably the MSI site is selected from one or more sites of Z1 ~ Z10.

In another preferred example, the kit is used to detect MSI sites in human DNA samples from tumor cells, tumor tissue, or suspected tumor tissue.

In another preferred example, the product is used for detecting cell-free DNA(cfDNA) samples.

In another preferred example, the cfDNA is derived from blood, plasma, or serum of a subject.

In another preferred embodiment, the subject is a tumor patient.

In another preferred embodiment, the tumor is selected from the following groups: colorectal cancer, endometrial cancer, uterine sarcoma, gastric cancer, small intestine cancer, cervical cancer, liver cancer, esophageal cancer, pancreatic cancer, ovarian cancer, gallbladder cancer, testicular cancer, prostate cancer, fallopian tube cancer, vulvar cancer, adrenal cortex cancer, primary abdominal tumor, cholangiocarcinoma, breast cancer, neuroendocrine tumor, thymic cancer, thyroid cancer, small cell lung cancer, primary unknown tumor, etc.

In the fourth aspect of the invention, it is provided a combination of detection reagents, and the combination of detection reagents comprising n detection reagents for detecting human microsatellite instability (MSI) sites, wherein the MSI site is selected from the site of group A, and n is a positive integer of ≥2.

Preferably, n is 2-25, and more preferably, n is 2, 3, 4, 5, 6, 7, 8, 9, 10, 11, 12, 13, 14, 15, 16, 17, 18, 19, 20, 21, 22, or 23.

In another preferred example, the MSI site is selected from one or more sites of group A (i.e., one or more of Z1 ~ Z23), and preferably the MSI site is selected from one or more sites of Z1 ~ Z10.

In another preferred example, the detection reagent is selected from the following group: a primer, a probes, a chip, and a combination thereof.

In another preferred example, the combination of detection reagents is a combination of detection reagents for detecting MSI sites of Z2, Z3, Z4, Z5 and Z7.

In a fifth aspect of the invention, it provides a method for detecting MSI in the sample to be tested, comprising steps:
(S1) A PCR reaction system is provided, the PCR reaction system comprises samples to be tested as templates and primer pairs for amplification, and the primer pairs are selected from the following group:
   (a) The first primer pair for detecting MSI at chr3 site 1(SEQ ID NO.:1), where the first primer pair comprises primers shown in SEQ ID NO.: 11 and 12;
   (b) The second primer pair for detecting MSI at chr11 site 2(SEQ ID NO.:2), in which the second primer pair comprises primers shown in SEQ ID NO.: 19 and 20;
   (c) The third primer pair for detecting MSI at chr16 site 3 (SEQ ID NO.:3), in which the third primer pair comprises primers shown in SEQ ID NO.:23 and 24;
   (d) The fourth primer pair for detecting MSI at chr17 site 4 (SEQ ID NO.:4), where the fourth primer pair comprises the primers shown in SEQ ID NO.:25 and 26;
   (e) The fifth primer pair for detecting MSI at chr20 site 5(SEQ ID NO.:5), where the fifth primer pair comprises primers shown in SEQ ID NO.:29 and 30;
   (f) The sixth primer pair for detecting MSI at chr2 site 6(SEQ ID NO.:6), where the sixth primer pair comprises primers shown in SEQ ID NO.: 13 and 14;
   (g) The seventh primer pair for detecting MSI at chr2 site 7(SEQ ID NO.:7), where the seventh primer pair comprises primers shown in SEQ ID NO.: 15 and 16;
   (h) The eighth primer pair for detecting MSI at chr6 site 8 (SEQ ID NO.:8), where the eighth primer pair comprises the primers shown in SEQ ID NO.: 17 and 18;
   (i) The ninth primer pair for detecting MSI at chr14 site 9 (SEQ ID NO.:9), where the fourth primer pair comprises the primers shown in SEQ ID NO.:21 and 22;
   (j) The tenth primer pair for detecting MSI at chr20 site 10(SEQ ID NO.: 10), where the tenth primer pair comprises primers shown in SEQ ID NO.:27 and 28;
   any combination of (a) to (j) above.
(S2) the PCR reaction system of step (S 1) is subjected to PCR reaction so as to obtain an amplification product;
(S3) The amplification product generated in step (S2) is analyzed to obtain the MSI of the sample to be tested; wherein taking the total number of detected sites P =5 as an example,
If the number of MSI sites in the sample P_{MSI} ≥2, it is judged to be highly unstable (MSI-H);
If the number of MSI sites in the sample P_{MSI} =1, it is judged to be lowly unstable (MSI-L);
If the number of MSI sites in the sample PMSI =0, it is judged to be stable (MSS).

In another preferred embodiment, if the total number of detected sites P ≥ 5, the percentage of MSI sites in the sample is P (PMSI/P total),
If P ≥40%, it is judged to be highly unstable (MSI-H);
If 10≤P <40%, it is judged to be low instability (MSI-L);
If P = 0, it is judged to be stable (MSS).

In another preferred embodiment, the analysis is qualitative, quantitative or semi -quantitative.

In another preferred embodiment, the reaction system is a sequencing reaction system, including a first, second, and third generation sequencing reaction system.

In another preferred embodiment, the PCR reaction system is a digital PCR reaction system.

In another preferred embodiment, the digital PCR is ddPCR.

In another preferred embodiment, the method is an *in vitro* method.

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Description of the figures

Figures 1A-1H show the electrophoresis results of PCR of 40 sites in DNA of 293T and HCT116 cells using primers (M: DNA Marker).
Figures 2A-2E show the two-dimensional digital PCR maps of the screened five sites, wherein FigureA a the two-dimensional digital PCR map of SEQ ID NO.:1. FigureB is a two-dimensional digital PCR map of SEQ ID NO.:2; Figure C is a two-dimensional digital PCR map of SEQ ID NO.:3; FigureD is a two-dimensional digital PCR map of SEQ ID NO.:4; and Figure E is a two-dimensional digital PCR map of SEQ ID NO.:5. Among them, gray is the unamplified fragment, green is the positive signal, and orange is the negative signal.
Figures 3A-3E show two-dimensional digital PCR maps for the detection of MSI in HCT-116 cell line; wherein Figure A is Site 1(SEQ ID NO.:1); Figure B is Site 2(SEQ ID NO.:2); Figure C is Site 3(SEQ ID NO.:3); Figure D is Site 4(SEQ ID NO.:4); and Figure E is site 5(SEQ ID NO.:5).
Figures 4A-4E show two-dimensional digital PCR maps for the detection of MSI in RKO cell line; wherein Figure A is Site 1(SEQ ID NO.:1); B is Site 2(SEQ ID NO.:2); C is Site 3(SEQ ID NO.:3); D is Site 4(SEQ ID NO.:4); and E is Site 5(SEQ ID NO.:5).
Figures 5A-5E show two-dimensional digital PCR maps for the detection of MSI in SW48 cell line; wherein Figure A is Site 1(SEQ ID NO.:1); Figure B is Site 2(SEQ ID NO.:2); Figure C is Site 3(SEQ ID NO.:3); Figure D is Site 4(SEQ ID NO.:4); and Figure E is Site 5(SEQ ID NO.:5).
Figure 6A shows the MSI detection results of case SY020 by capillary electrophoresis using human microsatellite instability detection kit 2B3D (BAT-25, BAT-26, D5S346, D17S250, D2S123).
Figure 6B shows the MSI detection results for new microsatellites (Z2, Z3, Z4, Z5 and Z7) of case SY020 by capillary electrophoresis.
Figure 7A shows the MSI detection results of case SY028 by capillary electrophoresis using human microsatellite instability detection kit 2B3D (BAT-25, BAT-26, D5S346, D17S250, D2S123).
Figure 7B shows the MSI detection results for new microsatellites (Z2, Z3, Z4, Z5 and Z7) of case SY028 by capillary electrophoresis.
Figure 8A shows the MSI detection results for new microsatellites (Z2, Z3, Z4, Z5 and Z7) of case SY020 by digital PCR.
Figure 8B shows the MSI detection results new microsatellites (Z2, Z3, Z4, Z5 and Z7) of case SY028 by digital PCR.
Figure 9A-9B show a two-dimensional digital PCR maps of the MSI detection results of case SY029; wherein Figure A is the detection results of site 5 in blood sample of case SY029, and Figure B is the detecion results of site 5 of tissue sample from case SY029.

### Detailed description

After extensive and intensive research, the inventor identified several novel MSI sites for the first time through extensive screening. These novel MSI sites have been shown to be highly associated with MSI-related diseases, such as tumors such as colon cancer, and thus can be used to assist in diagnosis and/or prognosis of MSI-related diseases. In addition, the primers and/or probes based on these new MSI sites are also optimized by the inventor, and the detection methods such as digital PCR are combined to effectively improve the detection effects of MSI, and can be used in plasma/serum samples detection, thus breaking through the problems of low accuracy and low sensitivity of pathological tissue as the starting material in the existing gene mutation detection technology. On this basis, the present invention has been completed.

Specifically, in the invention, 40 microsatellite sites composed of single nucleotide repeats (polyA or polyT) in the human genome were selected, amplification primer pairs were separately designed to perform PCR amplification in colorectal cancer cell lines with unstable MSI. From the microsatellite sites screened in amplification results, 10 optimal microsatellite sites were further selected. Probes that could detect both stable and unstable microsatellite sites were designed. Primers and probes were optimized in sequence, and the optimized primers and probes were used for digital PCR, and the optimal conditions for low frequency detection were optimized. The method was verified in tumor tissue and blood samples of primary colorectal cancer patients. The MSI detection method of the invention has advantages, such as high specificity and sensitivity with quick and convenient operation, simple interpretation. It can not only accurately detect tissue and body fluid samples, but also treat plasma/serum and other difficult samples, which are used for MSI liquid biopsy of different samples.

### Terms

To make this disclosure easier to be understood, some terms are firstly defined. As used in this application, each of the following terms shall have the meaning given below unless expressly provided herein. Other definitions are stated throughout the application.

The term "about" may mean a value or composition within the acceptable range of error of a particular value or composition as determined by a general skilled person in the field, which will depend in part on how the value or composition is measured or determined. For example, as used herein, the expression "about 100" comprises all values between 99 and 101 (eg, 99.1, 99.2, 99.3, 99.4, etc.).

As used herein, the term "including" or "comprising (comprise)" may be open, semi-close, and close-ended. In other words, the term also includes "essentially consisting of" or "consisting of".

As used herein, the term "blood" may refer to plasma or serum, but does not include blood cells.

As used herein, the term "isolated" means that a substance is separated from its original environment (if it is a natural substance, the original environment is the natural environment). For example, polynucleotides and polypeptides in the natural state of living cells are not isolated and purified, but if the same polynucleotides or polypeptides are separated from other substances in the natural state, they are isolated and purified.

Sequence identity is determined by comparing two aligned sequences along a predetermined comparison window (which can be 50%, 60%, 70%, 80%, 90%, 95%, or 100% of the length of a reference nucleotide sequence or protein) and determining the number of locations where identical residues occur. Normally, this is expressed as a percentage. The measurement of sequence identity of nucleotide sequences is well known to a skilled person in this field.

### Microsatellite

Microsatellite is a short series of repeats, with each unit being 1-6bp in length. It is widely present in the genomes of prokaryotic and eukaryotic organisms and has high genetic stability. However, when the function of the cell's mismatch repair gene is abnormal, the number of repeat nucleotides of the daughter cell microsatellite can increase or decrease, resulting in a change in the length of the microsatellite, and such a phenomenon is called microsatellite stability (MSI).

There are many microsatellite sites in the human genome. In 1998, five sites was recommended by MSI International Research Collaboration for detection: BAT26, BAT25, D5S346, D2S123, and D17S250. Taking colorectal cancer as an example, about 15% of colorectal cancer occurs through the MSI route, of which about 3% are familial (Lynch syndrome) and 12% are sporadic. According to the number of microsatellite instabilities, colorectal cancer was classified into high-frequency MSI(MSI-H, with two or more sites changed), low-frequency MSI(MSI-L, with only one site changed), and microsatellite stable (MSS, with no site changed). MSI-L colorectal cancer is usually classified as MSS tumor type because there is no significant difference between MSI-L colorectal cancer and MSS tumor in clinical manifestations and pathological changes. Clinical studies have found that MSI-H colorectal cancer has a lower risk of metastasis, suggesting a better prognosis than MSS colorectal cancer. MSI-H colorectal cancer is not sensitive to 5-fluorouracil and cisplatin, but sensitive to irinotecan, and MSI colorectal cancer is relatively sensitive to radiotherapy.

The method of the present invention is used to detect the MSI condition of colorectal cancer patients. If two or more sites are changed in the detection result, it is judged as MSI-H, indicating that the prognosis of the patient is good, 5-fluorouracil and platinum-based treatment schemes are not suitable, and irinotecan and other chemotherapy drugs can be considered. Results of MSI-L(only one site changed) or MSS(no site changed) indicate that the prognosis of the patient may be poor, and do not exclude treatment schemes mainly based on 5-fluorouracil and platinum.

As used herein, a "new effective site" in the invention refers to the site that can be detected at low frequency in colorectal cancer cell line with MSI instability compared with human normal cells after PCR screening by designed primers among 40 single nucleotide repeat (polyA or polyT) site selected in human genome. In another preferred embodiment, the "new effective site" in MSI sites is selected from the group A of Table 4 of Example 1:
(Z1) chr3:30650236-30650508
(Z2) chr11:106739898-106740117
(Z3) chr16:18841298-18841518
(Z4) chr17:19411505-19411722
(Z5) chr20:62921533-62921750
(Z6) chr2:47408320-47408461
(Z7) chr2:147906719-147906938
(Z8) chr6:142407071-142407290
(Z9) chr14:93268657-93268877
(Z10) chr20:47779916-47780134
(Z11) chr7:143306180-143306440
(Z12) chr1:201819424-201819543
(Z13) chr1:201771449-201771558
(Z14) chr2:61827581-61827677
(Z15) chr2:147857500-147857584
(Z16) chr4:82821524-82821646
(Z17) chr5:172998578-172998712
(Z18) chr6:142337918-142338138
(Z19) chr14:93244766-93244900
(Z20) chr15:45593287-45593508
(Z21) chr15:33349068-33349160
(Z22) chr15:33764931-33765150
(Z23) chr16:18854033-18854164

### Digital PCR technology

Digital PCR technology is an absolute quantitative method for counting nucleic acid based on single molecule PCR. It is mainly used microfluidic or microdroplet methods which are popular research fields in analytical chemistry, and a large amount of diluted nucleic acid solution is dispersed into microreactors or microdroplets in the chip, with less than or equal to one nucleic acid template in each reactor. After such PCR cycles, the fluorescence signal of each microdroplet was analyzed after the amplification. The reactor with nucleic acid molecular template would have fluorescence signal, while the reactor without template would have no fluorescence signal. From the relative proportions and the volume of the reactor, the concentration of nucleic acid in the original solution can be deduced.

Compared with conventional qPCR, digital PCR can accurately analyze and detect target nucleic acid molecules with high sensitivity. The conventional method for analyzing the results of qPCR is an analog method, whererin the digital PCR method, results of which are analyzed by digital method (as the obtained signal has the value of "0" or "1"), and has the advantages of analyzing samples of large volumes, detecting different samples at the same time and conducting different detections at the same time. Digital PCR is a technology that allows absolute quantification of DNA samples using single-molecule counting without a standard curve, and enables more precise absolute quantification of individual droplets per well by PCR (see Gudrun Pohl and le-Ming Shih, Principle and applications of digital PCR, Expert Rev. Mol.Diagn.4 (1), 41-47 (2004). Digital PCR has the advantages of high sensitivity, accurate quantification without standard curve and simple operation.

In digital PCR, each droplet containing sample gene templates, amplification primers, and fluorescent probes prepared and diluted to an average copy number of 0.5-1 are distributed into a single well, and microemulsion PCR is performed. Then, the well displaying a fluorescence signal is counted as the value "1", because the sample with 1 gene copy number is assigned to the well and after amplification, the well shows a fluorescence signal. While the well without a signal is counted as "0", because the sample with 0 gene copy number is assigned to the well and does not show a fluorescence signal due to no amplification. In this way, absolute quantification can be achieved.

### Modification of primers and probes

In the present invention, the nucleic acid sequence of the primer includes an unmodified or modified primer sequence.

Preferably, by modifying a probe with a locked-nucleotide, the specificity of the probe can be significantly improved, thus improving the sensitivity and specificity of the detection results.

In a preferred example of the invention, the mode of modification is selected from: Phosphorylation, Biotin, Digoxigenin, internal amino modification, 5' amino modification, 3' amino modification, Thiol, Spacer, Phosphorthioate, DeoxyUridine (dU), deoxyInosine (dI), or a combination thereof.

Phosphorylation modification: 5' phosphorylation can be used for coupling, cloning and gene construction, and linking reaction catalyzed by ligase. In the related experiments of 3' phosphorylation which is resistant to 3' exonuclease digestion, it is also used to block DNA strand elongation reactions catalyzed by DNA polymerase.

Biotin modification: Primer biotin labeling, which can be used for non-radioactive immunoassay to detect proteins, intracellular chemical staining, cell isolation, nucleic acid isolation, hybridization to detect specific DNA/RNA sequences, ion channel conformational changes, etc.

Digoxigenin modification: Digoxigenin is connected to the C5 position of uracil via an 11-atom interarm. The hybrid Digoxigenin probe can be detected by anti-Digoxigenin antibody. Digoxigenin labeled probes can be used for a variety of hybridization reactions. Examples include DNA-DNA hybridization (Southern blotting), DNA-RNA hybridization (Northern blotting), Dot blotting, clonal hybridization, in situ hybridization, and enzyme-linked immunoassay (ELISA).

Internal amino modification: C6-dT aminolinker is mainly used to add thymine residues for internal modification. The modified amino group is 10 atomic distances away from the main chain and can be used for further labeling and enzyme linking (such as alkaline phosphatase), which currently provide internal amino modification mediated dT-Dabcyl, dT-Biotin, and dT-Digoxingenin modifications.

5' amino modification: it can be used to prepare functionalized oligonucleotides and is widely used in DNA Microarray and multi-marker diagnostic systems. The 5' C6 amino modification and 5' C12 amino modification are currently available. The former can be used to bind compounds that do not affect their function even in proximity to oligonucleotides, and the latter is used to bind affinity purification groups and some fluorescent labels, especially when the fluorescence may be quenched when the labels are too close to the DNA strand.

3' amino modification: Currently 3' C6 amino modification is available. It can be used to design new diagnostic probes and antisense nucleotides, for example the 5' end can be used with highly sensitive 32P or fluorescence labels while the 3' end can be used with amino modification for other connections. In addition, 3' modification can inhibit the enzymolysis of 3' exonuclease, so it can be used in antisense experiments.

Sulfhydryl modification: 5' -sulfhydryl is similar in many respects to amino modification. Sulfhydryl can be used to attach various modifications such as fluorescent labels and biotin. For example, Sulfhydryl-linked fluorescent probes can be made in the presence of iodoacetic acid and maleimide derivatives. The sulfhydryl modification of 5' is mainly made using 5' sulfhydryl modified monomer (5'-Thiol-Modifier C6-CE Phosphoramidite or Thiol-Modifier C6 S-S CE Phosphoramidite). After 5'-Thiol-Modifier C6-CE monomer modification, silver nitrate oxidation must be performed to remove the protective base (trityl), and after Thiol-Modifier C6 S-S CE monomer modification, DTT must be used to reduce the disulfide bond to sulfhydryl.

Spacer modification: Spacer can provide the necessary interval for oligonucleotide labeling to reduce the interaction between labeling groups and oligonucleotide, and is mainly used in the study of DNA hairpin structure and double-stranded structure. C3 spacer is used primarily to mimic the three-carbon separation between the 3' and 5' hydroxyl groups of ribose, or to "replace" an unknown base in a sequence. 3'-Spacer C3 is used to introduce a 3' interarm that prevents 3' exonuclease and 3' end polymerase from acting. Spacer 18 is often used to introduce a strong hydrophilic group.

Thio-modification: Thio-modified oligonucleotides are mainly used in antisense experiments to prevent nuclease degradation. Total thiotide can be selected, however, the Tm value of oligonucleotide will decrease with the increase of thiotide bases. To reduce this effect, 2-5 bases of the two ends of the primer can be subjected to thio-modification. Usually, 3 bases of 5' and 3' can be selected for thiomodification.

Deoxyuracil modification: Deoxyuracil can be inserted into oligonucleotides to increase the melting point temperature of the double chain and thus increase the stability of the double chain. Each deoxythymine replaced by deoxyureacil can increase the double melting point temperature by 1.7°C.

DeoxyInosine modification: DeoxyInosine is a naturally occurring base that, although not a truly universal base, is relatively more stable than other base mismatches when combined with other bases. The binding capacity of deoxyInosine with other bases is dI:dC > dI:dA > dI:dG > dI:dT. DeoxyInosine is preferred to bind to dC when catalyzed by DNA polymerase,

### cfDNA

The free nucleic acid (Circulating free DNA, cfDNA) in plasma, also known as the "liquid biopsy," avoids the need for a biopsy of tumor tissue and is a useful diagnostic application in the clinic. The use of liquid biopsies provides the possibility of repeated blood collection, which allows changes in cfDNA to be tracked during tumor development or during cancer treatment, thereby monitoring Cell-free acids as biomarkers in cancer patients. However, the application of cfDNA to accurately and specifically detect gene mutations is a great technical challenge. Firstly, the amount of cfDNA Circulating in the blood varies from person to person and in many cases is very low. The free nucleic acid (ctDNA) from the tumor is of varying quality and circulating levels. Moreover, the specificity of cfDNA detection method needs to be improved. Douillard et al reported that plasma detection of EGFR mutations had only a 65% agreement with tumor tissue detection.

### Detection and judgment standard

Whether the microsatellite site is changed can be determined by the absolute quantitative method of the present invention by the two-dimensional map of fluorescence (Table 1), and whether it is MSI-H is determined according to the number of changes (Table 2). The detection and judgment standard of MSI of the present invention are shown in the following table:

**Table 1. Detection result table**

| | | |
|---|---|---|
| 1 | Positive droplets≥3, and the clustering is consistent with the positive control | Positive results |
| 2 | No positive droplets, and the clustering is consistent with the negative control | Negative results |
| 3 | Positive droplet(s) is 1 or 2 | Gray area, retest is recommended. If there are still less than 3 positive droplets, it is judged as negative |

**Table 2. MSI determination (taking 5 sites as examples)**

| | | | |
|---|---|---|---|
| 1 | ≥2 sites arepositive | MSI-H | ≥40% |
| 2 | 1 site is positive | MSI-L | 20-40% |
| 3 | 0 site is positive | MSS | 0 |

In the present invention, for example, when ≥5 sites are used and the percentage P of positive sites is 0, it can be judged as MSS; When the percentage P is 10-40%, it can be judged as MSI-L. When the percentage P is ≥ 40%, it can be judged as as MSI-H.

The main advantages of the invention include
1. High sensitivity: Since a digital PCR platform is used in the method, reaction system can be divided into 20,000 or more micro-reactions. Theoretically, it can detect the changes of a single copy, which has the advantage of sensitivity unmatched by other technologies. The existing PCR methods based on MSI detection generally require tumor tissue samples. Due to the excellent sensitivity of digital PCR, it is possible to detect MSI carried by small amounts of tumor DNA in cfDNA.
2. Strong specificity: The designed primers can target separately the sequences of microsatellite site detected by MSI, and specifically amplify stable microsatellites and unstable micro satellites. Among the designed primers, one probe (P1) covered the stable microsatellite site, and the other probe (P2) covered the sequence near the microsatellite site. The 5' end of P1 probe was modified by fluorescent groups such as FAM, Cy5, Texas Red or ROX, and the 5' end of P2 probe was modified by HEX or Vic groups.
3. Flexible requirements on the type and quality of samples and strong anti-interference strength: due to the characteristics of high sensitivity, the sample types applicable to the invention are not only fresh tissue samples and paraffin sections commonly used in general methods, but also peripheral blood samples; Moreover, due to the uniqueness of its digital PCR platform, the reaction system can be divided into about 20,000 or more small systems, and the interfering substances can be divided into the same number, which can greatly reduce the influence of interfering substances on the reaction, of course, it can also detect samples of more complex backgrounds. That's something that other platforms cannot achieve.
4. The positive interpretation method is simple: the invention obtains the final test result at the end of PCR reaction, which has incomparable timeliness compared with other current MSI detection methods.
5. Low cost: Although MSI can be detected by Next generation sequencing (NGS) in the current tumor liquid biopsy, the depth of NGS sequencing greatly exceeds the depth of tumor tissue source DNA due to the overall low ctDNA concentration in the blood, directly leading to an increase in sequencing cost. Moreover, errors of sequencing begin to appear in the process of deep sequencing, so complex pre-processing and bioinformatics analysis are needed to get the correct sequencing information, which makes it take a long time to get the test report. MSI detection based on ddPCR, however, does not have such problems. Generally, the detection can be completed within two hours, and the results can be obtained at the same time without complicated result analysis, which greatly reduces the waiting time of tumor patients and enables them to take drugs as soon as possible.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989, or as instructed by the manufacturers, unless otherwise specified. Unless otherwise stated, percentages and parts are by weight.

### Sequence of primers, probes, and sites

In the embodiments, the chromosome positions of the sequences are based on hg38, and the nucleic acid sequence information of the used primers, probes and DNA sites is shown in Table 3:

**Table 3. Nucleic acid sequences of primers, probes and DNA sites**

| **Name** | | **Sequence (5'-3')** | **SEQ ID NO.:** |
|---|---|---|---|
| Site 1 | chr3:3065 0236-306 50508 | CTCCCCTCGCTTCCAATGAATCTCTTCACTCTAGGAG AAAGAATGACGAGAACATAACACTAGAGACAGTTT GCCATGACCCCAAGCTCCCCTACCATGACTTTATTCT GGAAGATGCTGCTTCTCCAAAGTGCATTATGAAGGA AAAAAAAAAGCCTGGTGAGACTTTCTTCATGTGTTC CTGTAGCTCTGATGAGTGCAATGACAACATCATCTT CTCAGAAGGTGAGTTTTCTTCTCTTAAGGGTGTGGG ACCTGAGATCTGTGCCAATT | 1 |
| Site 2 | chr11:106 739898-1 06740117 | CCAGAGTTTAGCTCCTCAGAGGTCCCTAGCAGCACG ACTGACATAGCATCTTTCTAGTCTGCCGTAAGTACC ACTACCTTTACACCTCATTGAGAGGCACTTTTTTTTT TTTTTTTTTTTGAGATGGAGTCTTGCTCTGTCACCCC GGCTGCAGTGCAATGGCACATCTCAGCTCACTGCAA CCTCCGCCTCCCAGGTTCAAGTGATTCTCCTGCCTCA G | 2 |
| Site 3 | chr16:188 41298-18 841518 | GCCTGTAGCAGAATTGCTTGAACCTGGGAGGCAGAA GTTGCAGTGACCCAAAACCACACCACTGCACTCCAG CATGGGCGACAGAGCAAGACTGTGTCTCAAAAAAA AAAAAAAAAAAAAAGTACCTCAAAAATACTCTCCT AGGGACCTCAAGTCTTTTCCTTCTTTAACTGCTTATA AACATGCAAAAATGGTCCAGTAAGTCTACAAGTATT TCACAT | 3 |
| Site 4 | chr17:194 11505-19 411722 | AAGGGCCCTCCTTGGGCTGGGGGATGGGCCGGGGG TGGGGAGGATGAGGGGTCCCTGGAAATCCAGGGTG AAGATAGAAGTCTGGATTATGTTCTAAATCTTTTTTT | 4 |
| | | TTTTTTTTTTCTCCAAGGAGAGAAAACAGAGCTTC ATGGGAAACAGCGGCAACAGTTGGTAAGTAGCAGG GCCTTCCAGGCTGGGATGGGTGCAGTGAGGCCAGGC AGGT | |
| Site 5 | chr20:629 21533-62 921750 | AGATGATGGCCACCCTAAGTTCAGGTGGAGAGAAT GGGGTTTCTTCTTTAAAAACCCTTCTCTCACAAGAAT TTGCTTCTCAAATTGGAGCTGCAGCCAATTTTTTTTT TTTTTTTTTGGAGACAGGGTCTCTGTCGCCCAGGCTG GAGGGCAATGGAGCGACTGATCACGGCTCACTGCA GCCTCAATCTCCCTGGGCTCAGGTTATCCTTCTACCT | 5 |
| Site 6 | chr2:4740 8320-474 08461 | ATCAGCAGCATGAAGtccagctaatacagtgcttgaacatgtaatatctc aaatctgtaatgtactttttttttttttaaggagcaaagaatctgcagagtgttgtgcttag taaaatGAATTTTGAATCTTTTGTAAAAGA | 6 |
| Site 7 | chr2: 1479 06719-14 7906938 | CATTTTGAGTATTATATCAGGCACTATACAGATCAC TTTACACATATTAACTCATTTAATTTGTTAGTACCAT GGGGTTCTGTCCTGTTTATCAAGTCCATTTTTTTTTT TTTTTTTTTTTAACTTTAAATTCTGGGATACATGTGCA GAACATGCAGGTTTGTTACATAGGTATATCTGTGCC ATGGTGGTATGCTGCACCTATCAACCCGTCAGCTAG | 7 |
| Site 8 | chr6: 1424 07071-14 2407290 | GTGCCTGTAATCCTAGCACTTTGGGAGGCTGAGGAG GGAGGATTGCTTGAGACCAAGAGTTCAAGGCCAATC AAGCCAACATAGTGAGACCCGTCTCTTAAAAAAAA AAAAAAAAAAAAAGGAAGCATATGTTCAGTTTCTAA AGTTAATTTACAGGAGAATGAAAAAACTTTAAAGGT TCTGTGGTGCTCCCCTATCATAGAGGGCAAGGTACG ATGGT | 8 |
| Site 9 | chrl4:932 68657-93 268877 | AGGTTATACATCTAGGAAGAAACAAGTCCTACCTAA ACATATCCTTCCTTCCCCTCCCAAAAGTTCCCCTTCC CAGCCTTTTATGCCTCTAACTTAGACCTTTTTTTTTTT TTTTTTTTTTGAGACAGATTTTGCTCAGCCGCCCAGG CTGGAGTGCAGCAGCACGATCTCAGCTCACTACAAC CACCACCTCCTGGGTTCAAGGGATTCTCCCGTCTCA G | 9 |
| Site 10 | chr20:477 79916-47 780134 | TTTTATTTAAAATAAAGTCTAAAATTTCTTAGCATGG CCTGTATTTCTCATCTCTGACGTGCCCTCCCCCACTT ACTCCTGACCCTCTACCCTAGTTGACTTTTTTTTTTT TTTTTTTTGAGACAGAGTCTGACTCTGTTGCCCAGGCT GGAATGCAGAAGTGTGATCCCGGCTCACAGCAACCC CTGCCTCCCGGGTTCAAGTGATTCTCCTGTCTCA | 10 |
| Primer | Primer of Site 1 | AGTTTGCCATGACCCCAAGC | 11 |
| | | ACTCATCAGAGCTACAGGAACAC | 12 |
| | Primer of Site 2 | AGCACGACTGACATAGCATCT | 19 |
| | | GGGTGACAGAGCAAGACTCC | 20 |
| | Primer of Site 3 | GCAGTGACCCAAAACCACAC | 23 |
| | | ACTTGAGGTCCCTAGGAGAGT | 24 |
| | Primer of Site 4 | CCAGGGTGAAGATAGAAGTCTGG | 25 |
| | | ACTTACCAACTGTTGCCGCT | 26 |
| | Primer of Site 5 | AACCCTTCTCTCACAAGAATTTGC | 29 |
| | | GTGATCAGTCGCTCCATTGC | 30 |
| | Primer of Site 6 | TGAAGTCCAGCTAATACAGTGCT | 13 |
| | | ATTTTACTAAGCACAACACTCTGC | 14 |
| | Primer of Site 7 | CCATGGGGTTCTGTCCTGTT | 15 |
| | | CAGCATACCACCATGGCACA | 16 |
| | Primer of Site 8 | GCCAACATAGTGAGACCCGT | 17 |
| | | TGATAGGGGAGCACCACAGA | 18 |
| | Primer of Site 9 | TCCTTCCTTCCCCTCCCAAA | 21 |
| | | GCGGCTGAGCAAAATCTGTC | 22 |
| | Primer of Site 10 | CCTCCCCCACTTACTCCTGA | 27 |
| | | CCGGGATCACACTTCTGCAT | 28 |
| Probe | Probe of site 1 | CACCAGGCTTTTTTTTTTCCTTCA | 31 |
| | | CAGCATCTTCCAGAATAAAGTC | 32 |
| | Probe of site 2 | TTGAGAGGCACTTTTTTTTTTTTTTTTTTTTGAGATG | 39 |
| | | AGTCTTGCTCTGTCACC | 40 |
| | Probe of site 3 | TTGAGGTACTTTTTTTTTTTTTTTTTTTTTGAGACAC | 43 |
| | | TTGCTCTGTCGCCCATGCT | 44 |
| | Probe of site 4 | TCTAAATCTTTTTTTTTTTTTTTTTTCTCCAAGGAGAG | 45 |
| | | AAACAGAGCTTCATGGGAAAC | 46 |
| | Probe of site 5 | CAAATTGGAGCTGCAGCCAATTTTTTTTTTTTTTTTTTTT T | 49 |
| | | CCTGGGCGACAGAGACCC | 50 |
| | Probe of site 6 | TAATGTACTTTTTTTTTTTTTAAGG | 33 |
| | | CATGTAATATCTCAAATCT | 34 |
| | Probe of site 7 | TCAAGTCCATTTTTTTTTTTTTTTTTTTTTAACTTTA | 35 |
| | | ATACATGTGCAGAACATGCAGGT | 36 |
| | Probe of site 8 | ATGCTTCCTTTTTTTTTTTTTTTTTTTTAAAGAGACG | 37 |
| | | CATTCTCCTGTAAATTAAC | 38 |
| | Probe of site 9 | ACTTAGACCTTTTTTTTTTTTTTTTTTTTTGAGACAGA | 41 |
| | | TTGCTCAGCCGCCCAGG | 42 |
| | Probe of site 10 | CCCTAGTTGACTTTTTTTTTTTTTTTTTTTGAGACAGA | 47 |
| | | TGACTCTGTTGCCCAGGCTG | 48 |

| | | | |
|---|---|---|---|
| Note: All probes are fluorescent probes with a fluorescent group at one end and a quenching group at the other end. | | | |

### Example 1 screening of microsatellite sites

**Table 4. Screened microsatellite sites**

| **Number s** | **Site** | **Lengt h** | **Lane** | **Conclusio n** | **Note** |
|---|---|---|---|---|---|
| #1-1 | chr3:30650236-30650508 | 131 | Figure 1A-1,2 | + | Site 1 |
| #1-2 | chr2:47408320-47408461 | 108 | Figure 3A-3,4 | + | Site 6 |
| #1-3 | chr7:143306180-143306440 | 120 | Figure 1A-5,6 | + | |
| #1-4 | chr1:201677226-20167733 6 | 111 | Figure 1A-7,8 | - | |
| #1-5 | chr1:201819424-20181954 3 | 120 | Figure 1A-9,10 | + | |
| #1-6 | chr1:201771449-20177155 8 | 110 | Figure 1A-11,12 | + | |
| #1-7 | chr2:147906719-14790693 8 | 129 | (Figure 1B-13,14) | + | Site 7 |
| #1-8 | chr2:107849601-10784971 5 | 115 | Figure 1B-15,16 | - | |
| #1-9 | chr2:61827581-61827677 | 97 | Figure 1B-17,18 | + | |
| #1-10 | chr2:147857500-14785758 4 | 85 | Figure 1B-19,20 | + | |
| #1-11 | chr4:82849514-82849731 | 97 | Figure 1B-21,22 | - | |
| #1-12 | chr4:82820034-82820251 | 132 | Figure 1B-23,24 | - | |
| #1-13 | chr4:82821524-82821646 | 123 | Figure 1C-25,26 | + | |
| #1-14 | chr5:173027224-17302744 3 | 126 | Figure 1C-27,28 | - | |
| #1-15 | chr5:172998578-17299871 2 | 135 | Figure 1C-29,30 | + | |
| #1-16 | chr6:142337918-14233813 8 | 120 | Figure 1C-31,32 | + | |
| #1-17 | chr6:142394554-14239467 4 | 121 | Figure 1C-33,34 | - | |
| #1-18 | chr6:142407071-14240729 0 | 125 | Figure 1D-35,36 | + | Site 8 |
| #1-19 | chr7:1741030-1741248 | 132 | Figure 1D-37,38 | - | |
| #1-20 | chr7:1722166-1722384 | 135 | Figure 1D-39,40 | - | |
| #2-1 | chr11:106698028-1066981 62 | 135 | Figure 1E-1,2 | - | |
| #2-2 | chr11:106739898-1067401 17 | 115 | Figure 1E-3,4 | + | Site 2 |
| #2-3 | chr11:106777416-1067775 49 | 134 | Figure 1E-5,6 | - | |
| #2-4 | chr14:93244766-93244900 | 135 | Figure 1E-7,8 | + | |
| #2-5 | chr14:93268657-93268877 | 102 | Figure 1E-9,10 | + | Site 9 |
| #2-6 | chr14:93289781-93289901 | 121 | Figure 1E-11,12 | - | |
| #2-7 | chr15:33832903-33833121 | 130 | Figure 1E-13,14 | - | |
| #2-8 | chr15:45593287-45593508 | 117 | Figure 1F-15,16 | + | |
| #2-9 | chr15:33349068-33349160 | 93 | Figure 1F-17,18 | + | |
| #2-10 | chr15:33764931-33765150 | 103 | Figure 1F-19,20 | + | |
| #2-11 | chr16:18841298-18841518 | 116 | Figure 1F-21,22 | + | Site 3 |
| #2-12 | chr16:18854033-18854164 | 132 | Figure 1F-23,24 | + | |
| #2-13 | chr 16:18888721-18 888940 | 125 | Figure 1F-25,26 | - | |
| #2-14 | chr17:19411505-19411722 | 110 | Figure 1G-27,28 | + | Site 4 |
| #2-15 | chr20:47779916-47780134 | 110 | Figure 1G-29,30 | + | Site 10 |
| #2-16 | chr20:62921533-62921750 | 118 | Figure 1G-31,32 | + | Site 5 |
| #2-17 | chr20:47752536-47752622 | 87 | Figure 1G-33,34 | - | |
| #2-18 | chr22:29285033-29285166 | 134 | Figure 1G-35,36 | - | |
| #3-1 | chr 17:7423470-7423 603 | 134 | Figure 1H-1,2 | - | |
| #3-2 | chr12:6442426-6442543 | 118 | Figure 1H-3,4 | - | |

| | | | | | |
|---|---|---|---|---|---|
| Note: + indicates that there is an amplification and there is a difference in 2 cell lines, - indicates no difference or no amplification. | | | | | |

Among them, all new effective sites in Table 4 are in following Table A:

| Number s | Site | Lengt h | Lane | Conclusio n | Note |
|---|---|---|---|---|---|
| Z1 | chr3:30650236-30650508 | 131 | Figure 1A-1,2 | + | Site 1 |
| Z2 | chr11:106739898-1067401 17 | 115 | Figure 1E-3,4 | + | Site 2 |
| Z3 | chr16:18841298-18841518 | 116 | Figure 1F-21,22 | + | Site 3 |
| Z4 | chr17:19411505-19411722 | 110 | Figure 1G-27,28 | + | Site 4 |
| Z5 | chr20:62921533-62921750 | 118 | Figure 1G-31,32 | + | Site 5 |
| Z6 | chr2:47408320-47408461 | 108 | Figure 1A-3,4 | + | Site 6 |
| Z7 | chr2:147906719-147906938 | 129 | Figure 1B-13,14 | + | Site 7 |
| Z8 | chr6:142407071-14240729 | 125 | Figure 1D-35,36 | + | Site 8 |
| Z9 | chr14:93268657-93268877 | 102 | Figure 1E-9,10 | + | Site 9 |
| Z10 | chr20:47779916-47780134 | 110 | Figure 1G-29,30 | + | Site 10 |
| Z11 | chr7:143306180-14330644 0 | 120 | Figure 1A-5,6 | + | |
| Z12 | chr1:201819424-20181954 3 | 120 | Figure1A-9,10 | + | |
| Z13 | chr1:201771449-20177155 8 | 110 | Figure 1A-11,1 2 | + | |
| Z14 | chr2:61827581-61827677 | 97 | Figure 1B-17,18 | + | |
| Z15 | chr2:147857500-14785758 4 | 85 | Figure 1B-19,20 | + | |
| Z16 | chr4:82821524-82821646 | 123 | Figure 1C-25,26 | + | |
| Z17 | chr5:172998578-17299871 2 | 135 | Figure 1C-29,30 | + | |
| Z18 | chr6:142337918-14233813 8 | 120 | Figure 1C-31,32 | + | |
| Z19 | chr14:93244766-93244900 | 135 | Figure 1E-7,8 | + | |
| Z20 | chr15:45593287-45593508 | 117 | Figure 1F-15,16 | + | |
| Z21 | chr15:33349068-33349160 | 93 | Figure 1F-17,18 | + | |
| Z22 | chr15:33764931-33765150 | 103 | Figure 1F-19,20 | + | |
| Z23 | chr16:18854033-18854164 | 132 | Figure 1F-23,24 | + | |

As shown in Table 4, 40 single nucleotide repeat (polyA or polyT) sites were selected from the human genome and several pairs of primer pairs (primer synthesis from IDT Corporation of the United States) were designed for screening. Specific human genome loci and primer sequences are shown in Table 3.

Screening PCR system: Takara Taq HS Perfect Mix 10ul, primer 1 1ul, primer 2 1ul, human renal epithelial cell 293T or human colon cancer cell HCT116 cell genomic DNA template 5ul, water supplemented to 20ul. Screening PCR procedure: 32 cycles (94 °C 5s, 56 °C 1s, 68 °C 20s).

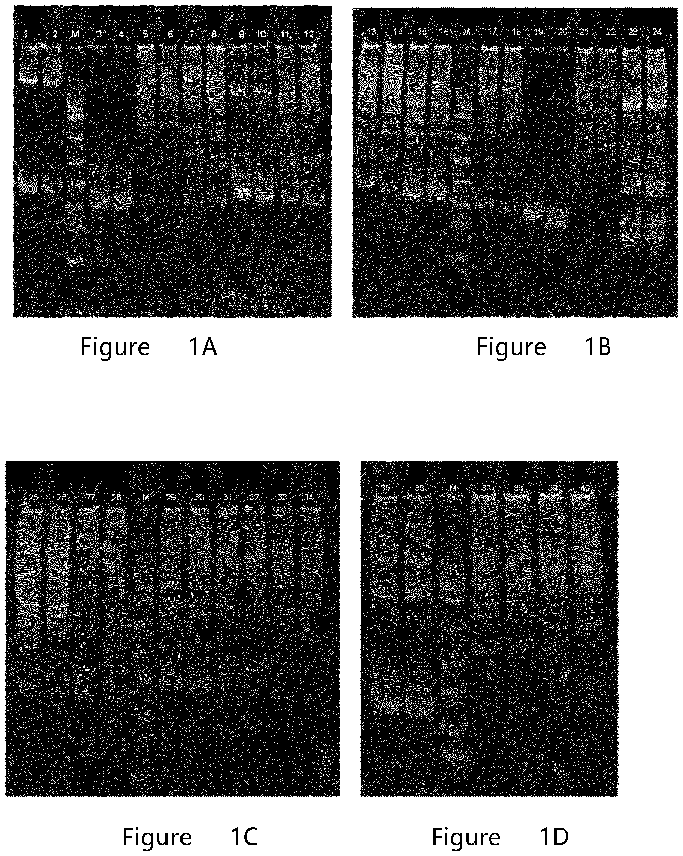

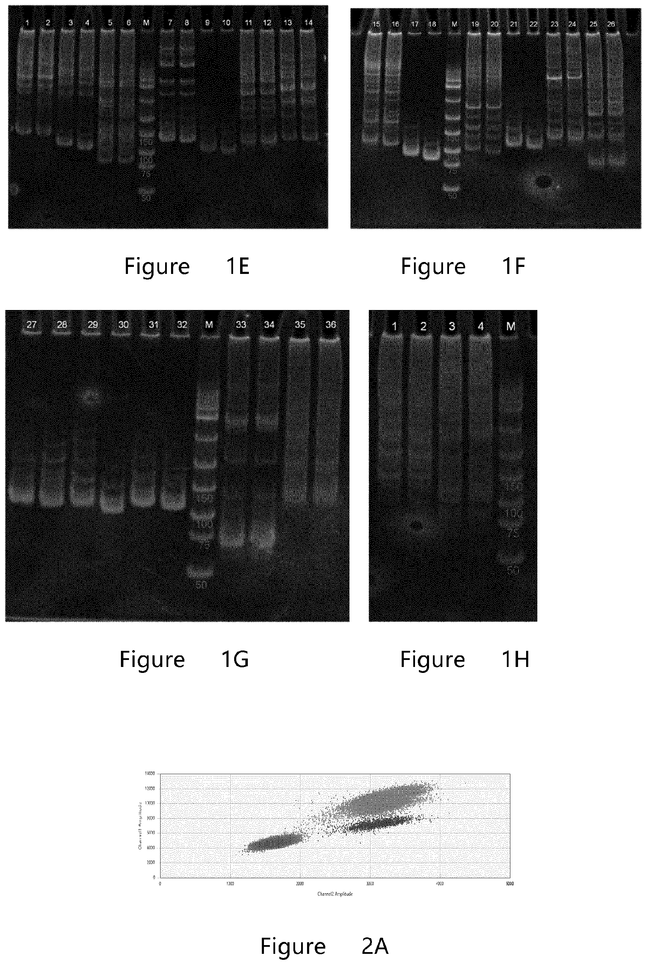

After PCR screening, the amplification efficiency of the final loci target fragment was relatively high, and the amplification products of 293T and HCT116 were different in size and had less heterobands, and the overall effect was the best. Some of these representative electrophoresis patterns are shown in Figure 1. The new effective sites of MSI with different electrophoresis results or amplification are shown in Z1-Z23 in Table A.

Based on the results of Figure 1, 10 sites (Z1-Z10) are preferred.

### Example 2 Digital PCR method to detect MSI method for screening

According to the sequence of 10 sites screened out in Example 1, probes were designed separately (the probes are synthesized from IDT Corporation of the United States), and the designed probe sequence is shown in Table 3. The probe were optimized by the following steps:
1. Configuration of PCR system. The PCR system was configured in the reagent preparation area according to the following table:

| | |
|---|---|
| 2×ddPCRSupermix(No dUTP) | 11ul |
| Primer 1(F) | 1.1ul |
| Primer 2(R) | 1.1ul |
| Probe 1(P1) | 0.55ul |
| Probe 2(P2) | 0.55ul |
| Template | 5.5ul |
| Add water to | 22ul |

2. Additon of templates. Templates were added in the sample preparation area in the following order: blank control, negative control (293T cell DNA), positive control (HCT116 cell DNA).
3. Generation of microdroplets. Droplet generation was carried out according to the requirements of the droplet generator.
4. PCR: PCR was performed according to the following procedure: 95°C for 10min, 40 cycles (94°C 30s, 56°C 30s, 72°C 30s), 98°C for 10min, ramp rate was 2°C/s.
5. Data Reading.

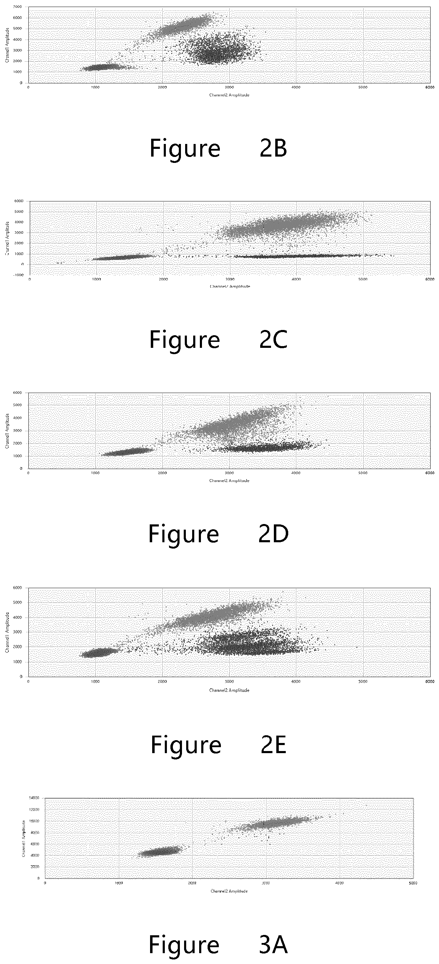

According to the experimental results, 5 sites were singled out, and the positive signal (only HEX signal, green) and negative signal (both FAM and HEX signal, orange) were well separated, and the digital PCR two-dimensional maps were shown in Figure 2A-2E. Among them, the orange signal is the negative droplet signal and the green signal is the positive (MSI) droplet signal.

### Example 3 Digital PCR method to detect MSI of tumor cell lines

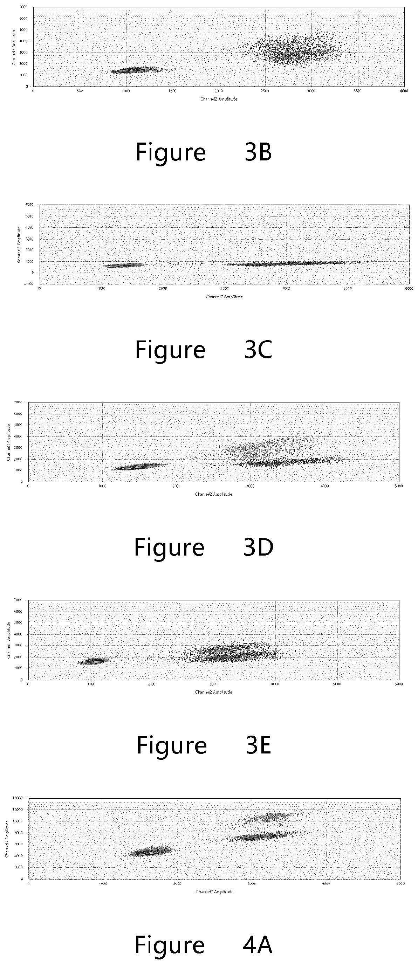

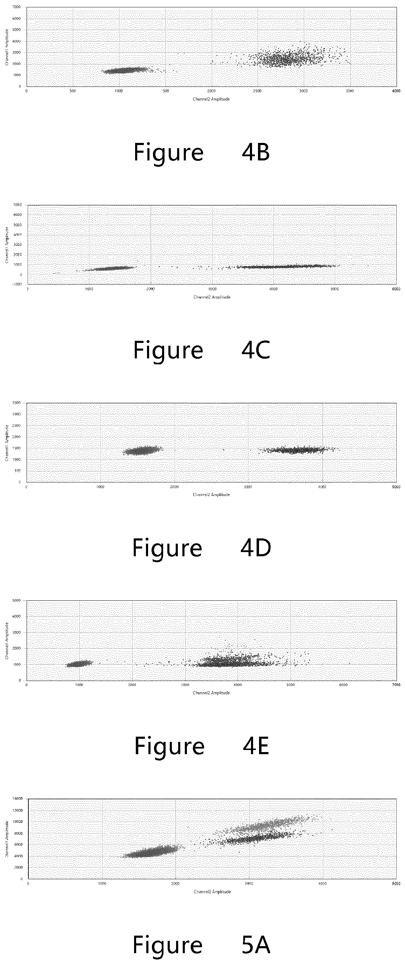

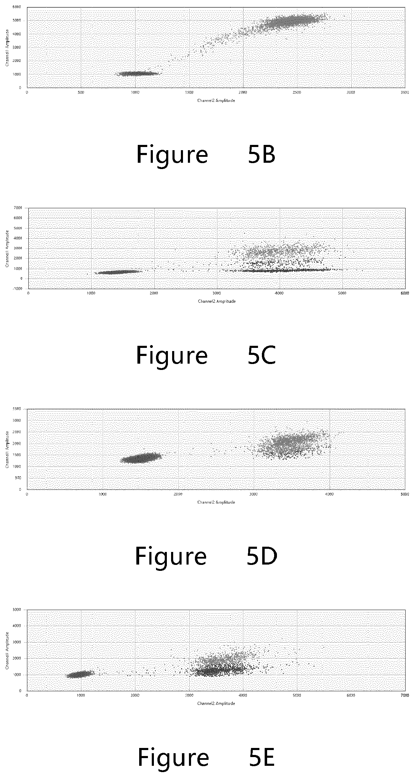

From Example 2, 5 sites (Z2, Z3, Z4, Z5 and Z7) were selected to detect the DNA of colorectal cancer cell lines HCT-116, RKO, SW48 by the method in Example 2, and HCT-116, RKO and SW48 were judged to be MSI-H. The digital PCR two-dimensional maps were shown in Figures 3-5, and the detection results were shown in Table 5.

Figures 3A-E were the HCT-116 cell detection results, Figure 3A shows only negative signal points (orange dots), which are judged to be MSS; Figures 3B, 3C, and 3E only have positive signal points (green dots), which are judged to be MSI; Figure 3D shows both negative and positive signal points, which are also determined to be MSI. Thus, for HCT-116 cells, 4 out of 5 sites are judged to be MSI, so it is judged as MSI-H as a whole.

The RKO and SW48 were determined in the same way.

**Table 5. MSI detection results of different sites in several rectal cancer cell lines**

| Rectal cancer cell line | HCT-116 | RKO | SW48 |
|---|---|---|---|
| Site 2(SEQ ID NO.:2) | MSS | MSI | MSI |
| Site 3(SEQ ID NO.:3) | MSI | MSI | MSS |
| Site 4(SEQ ID NO.:4) | MSI | MSI | MSI |
| Site 5(SEQ ID NO.:5) | MSI | MSI | MSI |
| Site 7(SEQ ID NO.:7) | MSI | MSI | MSI |
| Result judgment | MSI-H | MSI-H | MSI-H |

### Example 4 new site and 2B3D site compared by Capillary electrophoresis method

Cases "SY020" and "SY028" were colorectal cancer patients. Its FFPE specimens were extracted genomic nucleic acids. The extracted nucleic acids were quantified by Qubit and stored in a -20°C freezer in the sample preparation area.

The primer sequence in Example 2 was taken and labeled with a fluorophore at one end primer, the tumor specimen and the genomic nucleic acid of the adjacent tissue of SY020 and SY028 were amplified, respectively, and the results were detected by capillary electrophoresis.

The Human Microsatellite Instability Detection Kit (2B3D) was purchased from Shanghai Tongshu Biotechnology Co., Ltd., and the tumor samples and genomic nucleic acids of SY020 and SY028 tumor samples and adjacent tissues were amplified according to the instructions method, and the results were detected by capillary electrophoresis. This kit requires at least 20% tumor cells in the tumor specimen.

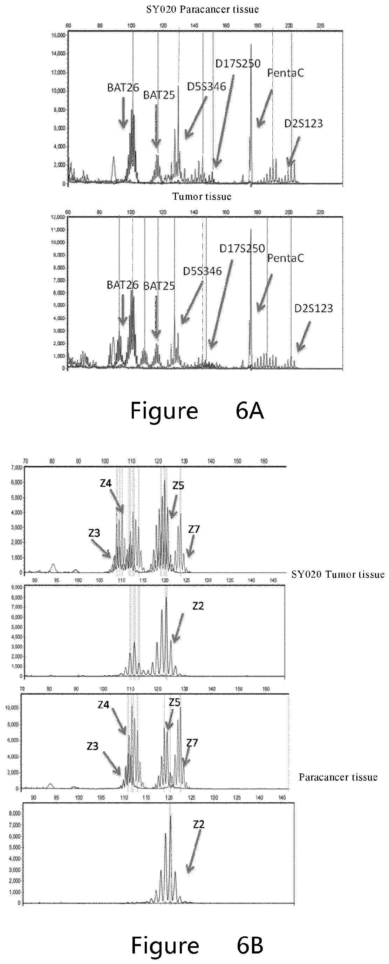

The results showed that 2 of the 5 sites of 2B3D of SY020 were positive (BAT-25, BAT-26) and 3 sites were negative (D5S346, D17S250, D2S123), which were judged to be MSI-H (see Figure 6A). All 5 sites of the new sites (Z2, Z3, Z4, Z5, and Z7) of SY020 were positive and were judged to be MSI-H (see Figure 6B).

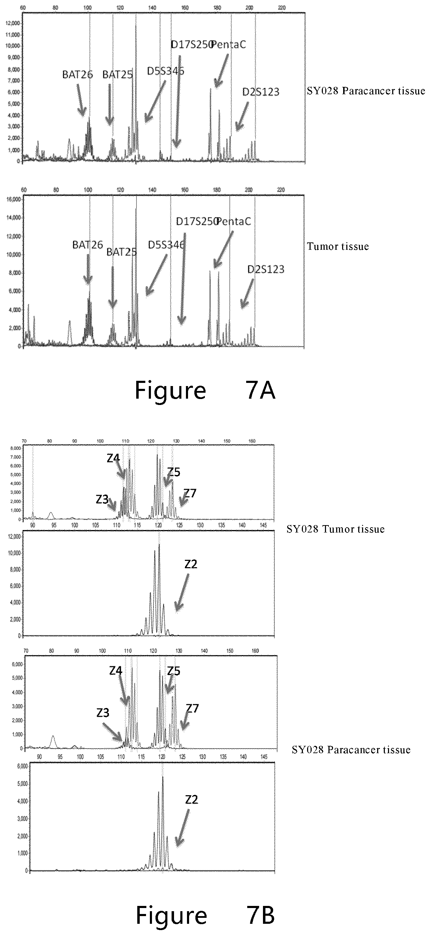

All 5 sites of 2B3D of SY028 were negative and judged as MSS (see Figure 7A), and 5 sites of the new sites (Z2, Z3, Z4, Z5, and Z7) were negative and judged as MSS (see Figure 7B).

A total of 263 colorectal cancer specimens were compared in this method, and the results are summarized in Table 6.

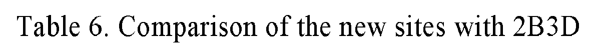

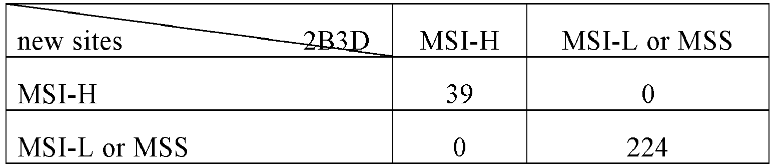

The results showed that the results of the new sites were comparable to those of 2B3D.

### Example 5 MSI detected by digital PCR method

From Example 2, 5 sites (Z2, Z3, Z4, Z5 and Z7) were selected to detect 263 cases of colorectal cancer tumor tissue using digital PCR by the method in Example 2.

According to the same method of Example 2, the PCR reaction system was generated into droplets. PCR was performed according to the optimized PCR procedure: 95°C for 10min, 40 cycles (94°C 30s, 56°C 15s, 72°C 15s), 98°C for 10min, ramp rate was 2°C/s. The instrument was turned on, and set up as required, and the plate was started to read.

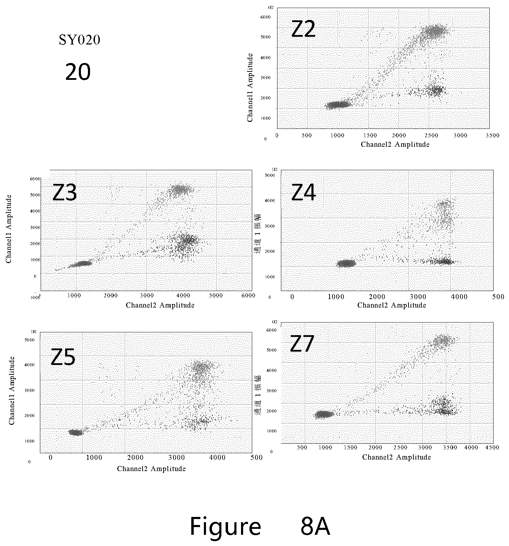

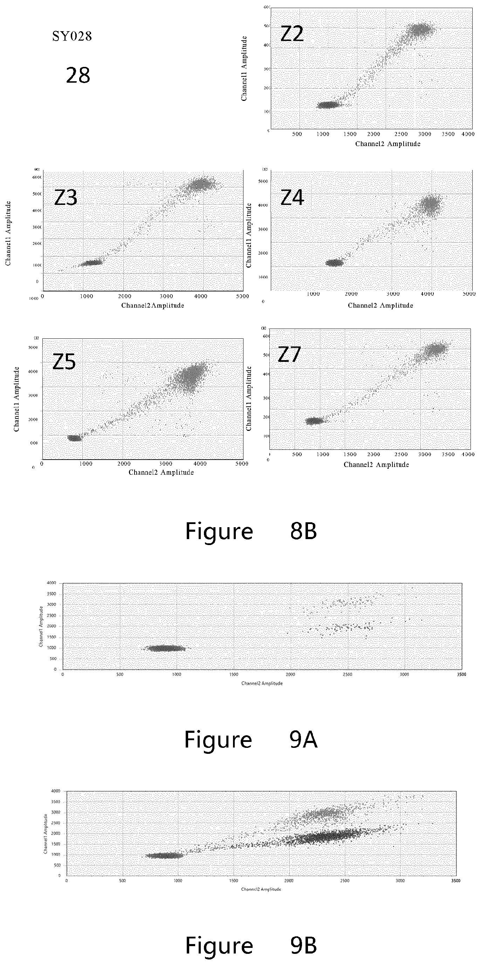

The results showed that the consistency between the digital PCR method and the capillary electrophoresis method was 100%. Taking SY020 (MSI-H) and SY028 (MSS) as examples, the digital PCR test results were shown in Figures 8A and 8B.

### Example 6 MSI detection based on blood samples

The blood sample of case "SY029" was collected in the Streck Cell-Free DNA Blood Collection Tube, with 10ml in total.

Plasma separation: first centrifugation, 4 °C, 1900×g, 10min. The plasma supernatant was carefully aspirated into a clean 15 ml centrifuge tube without touching the leukocyte layer; second high-speed centrifugation, 4 °C, 16000 ×g, 10min. The supernatant was carefully pipetted to a 50 ml centrifuge tube without touching the pellet. About 4.5 ml of plasma was separated.

Plasma free nucleic acid extraction: free nucleic acid was eluted with 50ul nuclease-free water by using Kangwei Century Free Nucleic Acid Extraction Kit, according to the instructions.

According to the method in Example 4, site 5 was detected, while the DNA of the tissue sample of case "SY029" was used as a control. The results showed that the presence of MSI in the tissue sample can also be detected in the blood. (See Figures 9A, 9B).
9A: The detection result of site 5 of the blood sample of case "SY029" was MSI, and the proportion of unstable sites was 46.7%;
9B: The detection result of site 5 of the tissue sample of case "SY029" was MSI, and the proportion of unstable sites was 71%;

### Discussion

The clinical significance of distinguishing MSI colorectal cancer includes the following three aspects:
1. Prognosis: Clinical studies have found that the proportion of MSI-H colorectal cancer in stage II colorectal cancer (22%) is higher than that of stage III colorectal cancer (12%), while only 3.5% in stage IV tumors. This suggests that MSI-H colorectal cancer has a low risk of metastasis. Especially in patients with stage II colorectal cancer, MSI status can be used as an independent predictor of prognosis: MSI-H tumors have a better prognosis than MSS tumors. Risk factors for recurrence of stage II colorectal cancer include poorly differentiated carcinoma, vascular/nerve invasion, fewer than 12 lymph nodes, obstruction or perforation, and positive margins in tumor histology. However, if the tumor is MSI-H type, poorly differentiated carcinoma is not a high-risk factor.
2. Guiding treatment: The study found that MSI-H colorectal cancer was not sensitive to chemotherapy drugs such as 5-fluorouracil and cisplatin, but to chemotherapy drugs such as irinotecan, and MSI colorectal cancer is more sensitive to radiotherapy. The National Comprehensive Cancer Network (NCCN) clinical practice guidelines for colorectal cancer clearly state that 5-fluorouracil and platinum-based regimens are not suitable for MSI-H colorectal cancer.
3. Screening of patients with Lynch syndrome: In patients with Lynch syndrome, simultaneous or metachronous multiple onsets of colorectal cancer are more common. If the diagnosis of Lynch syndrome can be confirmed before surgery, surgeons may have the option of more extensive resection. Therefore, MSI detection is extremely necessary for colorectal cancer patients.

At present, main methods of MSI detection are:
1. Immunohistochemical detection of mismatch repair protein: the loss of function of one or more mismatch repair proteins can cause MSI-H, therefore, detecting the loss of mismatch repair protein can indirectly reflect the MSI status of tumors. At present, the four main mismatch repair proteins MLH1, MSH2, MSH6 and PMS2 all have stable commercial antibodies, but due to different factors such as tissue fixation, staining conditions, antibody clone number, false positive or false negative results may occur. This method cannot be used for the testing of blood samples.
2. MSI detection based on conventional PCR: The principle of PCR detection of MSI is to amplify specific microsatellite sequences through PCR methods, and determine whether there is MSI phenomenon by comparing the difference in the length of microsatellite sequences between tumor tissue and normal tissue. Due to its limited sensitivity, this method is not suitable for the detection of blood samples, and generally requires the acquisition of normal and tumor tissues from patients. Generally, this method is carried out by two detection methods:
   (1) Capillary electrophoresis: By far, the most commonly used method is to use capillary electrophoresis to analyze the length distribution of marker amplification products for diagnosis. The most widely known analytical method of "multiplex fluorescence PCR amplification and capillary electrophoresis" is a method by extracting DNA from normal and tumor tissue, amplifying DNA by fluorescent PCR targeting satellite markers, and then analyzing the fluorescence of DNA using capillary electrophoresis to analyze MSI. However, this method requires an expensive capillary electrophoresis device to operate in two steps. There is the possibility of laboratory contamination in the process of adding samples after PCR, and it takes a long time. The results need to be analyzed by experienced and trained laboratory personnel.
   (2) Isotope labeling PCR method: Using the sensitivity of isotopes, PCR primers can be labeled with isotopes, and the length of PCR products can be detected by exposure after electrophoresis of the amplified PCR products. This method requires isotopic operation, which is harmful to human health. It is time-consuming, and requires special operation space and treatment of experimental waste, which may pollute the environment. So far it has been currently rare.
3. MSI detection based on next-generation sequencing (NGS): The principle of NGS detection of MSI is to compare the difference between the length of microsatellite sequences of tumor tissue and normal tissues by directly reading out specific microsatellite sequences to determine whether there is MSI phenomenon. Although NGS can be detected with MSI with blood samples, its cost and timeliness cannot be compared with that in digital PCR methods. In summary, the existing MSI detection technology is cumbersome to operate, time-consuming, highly subjective in positive interpretation, and has high requirements for the type and quality of samples, and most of them need to provide tissue samples.

In recent years, many studies have confirmed that the sensitivity and specificity of the microsatellite marker detection of single nucleotide repeats is higher than the microsatellite marker detection of double nucleotide repeats, and the 5 sites recommended by MSI International Research Cooperation are not satisfactory for digital PCR. So a new set of MSI sites is re-screened in the present invention, and a corresponding digital PCR detection method is developed. The method of detecting MSI of the present invention has the advantages of high sensitivity, accurate quantification without standard curve, and simple operation, etc.

All literatures mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. Use of a human Microsatellite Instability (MSI) site detection reagent in the preparation of a diagnostic reagent or kit for the diagnosis of MSI-related diseases and/or for the prognosis of MSI-related diseases;
wherein the MSI site is selected from one or more sites in the following group A:
(Z1) chr3:30650236-30650508;
(Z2) chr11:106739898-106740117;
(Z3) chr16:18841298-18841518;
(Z4) chr17:19411505-19411722;
(Z5) chr20:62921533-62921750;
(Z6) chr2:47408320-47408461;
(Z7) chr2:147906719-147906938;
(Z8) chr6:142407071-142407290;
(Z9) chr14:93268657-93268877;
(Z10) chr20:47779916-47780134;
(Z11) chr7:143306180-143306440;
(Z12) chr1:201819424-201819543;
(Z13) chr1:201771449-201771558;
(Z14) chr2:61827581-61827677;
(Z15) chr2:147857500-147857584;
(Z16) chr4:82821524-82821646;
(Z17) chr5:172998578-172998712;
(Z18) chr6:142337918-142338138;
(Z19) chr14:93244766-93244900;
(Z20) chr15:45593287-45593508;
(Z21) chr15:33349068-33349160;
(Z22) chr15:33764931-33765150;
(Z23) chr16:18854033-18854164_{∘}

2. The use of claim 1, whererin the MSI site is selected from the group consisting of:
(Z1) chr3:30650236-30650508;
(Z2) chr11:106739898-106740117;
(Z3) chr16:18841298-18841518;
(Z4) chr17:19411505-19411722;
(Z5) chr20:62921533-62921750;
(Z6) chr2:47408320-47408461;
(Z7) chr2:147906719-147906938;
(Z8) chr6:142407071-142407290;
(Z9) chr14:93268657-93268877;
(Z10) chr20:47779916-47780134;
(Y1) any combinations of Z1-Z10.

3. The use of claim 1, whererin the MSI site comprises (a) one or more (e.g., 2, 3, 4, or 5) sites selected from Z2, Z3, Z4, Z5 and Z7; and (b) additional MSI sites in addition to Z2, Z3, Z4, Z5 and Z7.

4. The use of claim 3, whererin the additional MSI site is selected from the following group: BAT-26, BAT-25, MONO-27, NR-21, NR-24, D5S346, D2S123, D17S250, and a combination thereof.

5. The use of claim 1, whererin the MSI-related disease is a tumor or cancer.

6. The use of claim 5, whererin the tumor or cancer is selected from the following group: colorectal cancer, endometrial cancer, uterine sarcoma, gastric cancer, small intestine cancer, cervical cancer, liver cancer, esophageal cancer, pancreatic cancer, ovarian cancer, gallbladder cancer, testicular cancer, prostate cancer, fallopian tube cancer, vulvar cancer, adrenal cortex cancer, primary abdominal tumor, cholangiocarcinoma, breast cancer, neuroendocrine tumor, thymic cancer, thyroid cancer, small cell lung cancer, primary unknown tumor, etc.

7. The use of claim 1, whererin the diagnostic reagent or kit is used for a detection selected from the following group: serum detection, plasma detection, cell detection, tissue sample detection.

8. The use of claim 1, whererin the detection is a PCR detection or sequencing detection; preferably, digital PCR (ddPCR) detection.

9. A reagent for the detection of human Microsatellite Instability (MSI) sites, wherein the sites is selected from sites 1 ~ 10 in chromosome hg38 (SEQ ID NO.: 1 ~ 10); wherein the reagent is selected from the following group:
(a) The first primer pair for detecting MSI at chr3 site 1(SEQ ID NO.:1), where the first primer pair comprises primers shown in SEQ ID NO.: 11 and 12;
(b) The second primer pair for detecting MSI at chr11 site 2(SEQ ID NO.:2), in which the second primer pair comprises primers shown in SEQ ID NO.: 19 and 20;
(c) The third primer pair for detecting MSI at chr16 site 3 (SEQ ID NO.:3), in which the third primer pair comprises primers shown in SEQ ID NO.:23 and 24;
(d) The fourth primer pair for detecting MSI at chr17 site 4 (SEQ ID NO.:4), where the fourth primer pair comprises the primers shown in SEQ ID NO.:25 and 26;
(e) The fifth primer pair for detecting MSI at chr20 site 5(SEQ ID NO.:5), where the fifth primer pair comprises primers shown in SEQ ID NO.:29 and 30;
(f) The sixth primer pair for detecting MSI at chr2 site 6(SEQ ID NO.:6), where the sixth primer pair comprises primers shown in SEQ ID NO.: 13 and 14;
(g) The seventh primer pair for detecting MSI at chr2 site 7(SEQ ID NO.:7), where the seventh primer pair comprises primers shown in SEQ ID NO.: 15 and 16;
(h) The eighth primer pair for detecting MSI at chr6 site 8 (SEQ ID NO.:8), where the eighth primer pair comprises the primers shown in SEQ ID NO.: 17 and 18;
(i) The ninth primer pair for detecting MSI at chr14 site 9 (SEQ ID NO.:9), where the fourth primer pair comprises the primers shown in SEQ ID NO.:21 and 22;
(j) The tenth primer pair for detecting MSI at chr20 site 10(SEQ ID NO.:10), where the tenth primer pair comprises primers shown in SEQ ID NO.:27 and 28;
any combinations of (a) to (j) above.

10. The reagent of claim 9, wherein the reagent further comprises:
(a1) The first probe used in combination with the first primer pair, wherein the first probe is selected from the following group: a probe as shown in SEQ ID NO.: 31, a probe as shown in SEQ ID NO.: 32 or a combination thereof; and/or
(b1) The second probe used in combination with the second primer pair, wherein the second probe is selected from the following group: a probe as shown in SEQ ID NO.: 39, a probe as shown in SEQ ID NO.: 40 or a combination thereof; and/or
(c1) The third probe used in combination with the third primer pair, wherein the third probe is selected from the following group: a probe as shown in SEQ ID NO.: 43, a probe as shown in SEQ ID NO.: 44 or a combination thereof; and/or
(d1) The fourth probe used in combination with the fourth primer pair, wherein the fourth probe is selected from the following group: a probe as shown in SEQ ID NO.: 45, a probe as shown in SEQ ID NO.: 46 or a combination thereof; and/or
(e1) The fifth probe used in combination with the fifth primer pair, wherein the fifth probe is selected from the following group: a probe as shown in SEQ ID NO.: 49, a probe as shown in SEQ ID NO.: 50 or a combination thereof; and/or
(f1) The sixth probe used in combination with the sixth primer pair, wherein the sixth probe is selected from the following group: a probe as shown in SEQ ID NO.: 33, a probe as shown in SEQ ID NO.: 34 or a combination thereof; and/or
(g1) The seventh probe used in combination with the seventh primer pair, wherein the seventh probe is selected from the following group: a probe as shown in SEQ ID NO.: 35, a probe as shown in SEQ ID NO.: 36 or a combination thereof; and/or
(h1) The eighth probe used in combination with the eighth primer pair, wherein the eighth probe is selected from the following group: a probe as shown in SEQ ID NO.: 37, a probe as shown in SEQ ID NO.: 38 or a combination thereof; and/or
(i1) The ninth probe used in combination with the ninth primer pair, wherein the ninth probe is selected from the following group: a probe as shown in SEQ ID NO.: 41, a probe as shown in SEQ ID NO.: 42 or a combination thereof; and/or
(j 1) The tenth probe used in combination with the tenth primer pair, wherein the tenth probe is selected from the following group: a probe as shown in SEQ ID NO.: 47, a probe as shown in SEQ ID NO.: 48 or a combination thereof; and/or

11. The reagent of claim 10, wherein the structure of the first probe to the tenth probe (5'-3') is shown in formula I:
L1-L2-L3 I
Wherein,
L1 is a fluorescence group and L3 is a quenching group; or L3 is the fluorescence group and L1 is a quenching group;
L2 is a specific complementary nucleic acid sequence of nucleotides;
"-" is a chemical bond, a connecting group, or a linker of one or three nucleotides.

12. A kit comprising the reagent for the detection ofg MSI sites of claim 9.

13. The kit of claim 12, wherein the MSI site is selected from one or more sites of group A (i.e., one or more of Z1 ~ Z23), and preferably the MSI site is selected from one or more sites of Z1 ~ Z10.

14. The kit of claim 12, the kit is used to detect MSI sites in human DNA samples from tumor cells, tumor tissue, or suspected tumor tissue.

15. A combination of detection reagents, and the combination of detection reagents comprising n detection reagents for detecting human microsatellite instability (MSI) sites, wherein the MSI site is selected from the site of group A, and n is a positive integer of ≥2; preferably, n is 2-25, and more preferably, n is 2, 3, 4, 5, 6, 7, 8, 9, 10, 11, 12, 13, 14, 15, 16, 17, 18, 19, 20, 21, 22, or 23.

16. A method for detecting MSI in the sample to be tested, comprising steps:
(S1) A PCR reaction system is provided, the PCR reaction system comprises samples to be tested as templates and primer pairs for amplification; and the primer pairs are selected from the following group:
(a) The first primer pair for detecting MSI at chr3 site 1(SEQ ID NO.:1), where the first primer pair comprises primers shown in SEQ ID NO.: 11 and 12;
(b) The second primer pair for detecting MSI at chr11 site 2(SEQ ID NO.:2), in which the second primer pair comprises primers shown in SEQ ID NO.:19 and 20;
(c) The third primer pair for detecting MSI at chr16 site 3 (SEQ ID NO.:3), in which the third primer pair comprises primers shown in SEQ ID NO.:23 and 24;
(d) The fourth primer pair for detecting MSI at chr17 site 4 (SEQ ID NO.:4), where the fourth primer pair comprises the primers shown in SEQ ID NO.:25 and 26;
(e) The fifth primer pair for detecting MSI at chr20 site 5(SEQ ID NO.:5), where the fifth primer pair comprises primers shown in SEQ ID NO.:29 and 30;
(f) The sixth primer pair for detecting MSI at chr2 site 6(SEQ ID NO.:6), where the sixth primer pair comprises primers shown in SEQ ID NO.:13 and 14;
(g) The seventh primer pair for detecting MSI at chr2 site 7(SEQ ID NO.:7), where the seventh primer pair comprises primers shown in SEQ ID NO.:15 and 16;
(h) The eighth primer pair for detecting MSI at chr6 site 8 (SEQ ID NO.:8), where the eighth primer pair comprises the primers shown in SEQ ID NO.:17 and 18;
(i) The ninth primer pair for detecting MSI at chr14 site 9 (SEQ ID NO.:9), where the fourth primer pair comprises the primers shown in SEQ ID NO.:21 and 22;
(j) The tenth primer pair for detecting MSI at chr20 site 10(SEQ ID NO.:10), where the tenth primer pair comprises primers shown in SEQ ID NO.:27 and 28; any combinations of (a) to (j) above;
(S2) the PCR reaction system of step (S 1) is subjected to PCR reaction to obtain an amplification product;
(S3) The amplification product generated in step (S2) is analyzed to obtain the MSI of the sample to be tested; wherein taking the total number of detected sites P =5 as an example,
If the number of MSI sites in the sample P_{MSI} ≥2, it is judged to be highly unstable (MSI-H);
If the number of MSI sites in the sample P_{MSI} =1, it is judged to be lowly unstable (MSI-L);
If the number of MSI sites in the sample PMSI =0, it is judged to be stable (MSS).

17. The method of claim 16, wherein if detected sites number Pₜₒₜₐₗ ≥ 5, the percentage of MSI sites in the sample is P (P_{MSI}/Pₜₒₜₐₗ),
If P ≥40%, it is judged to be highly unstable (MSI-H);
If 10≤P <40%, it is judged to be low instability (MSI-L);
If P = 0, it is judged to be stable (MSS).
